# EUROPÄISCHE PATENTSCHRIFT

(11) **EP 0 437 271 B1**
(45) Veröffentlichungstag und Bekanntmachung des Hinweises auf die Patenterteilung: **13.04.1994**
(21) Anmeldenummer: 91100294.7
(22) Anmeldetag: 11.01.1991
(51) Int. Cl.: A61C 19/00, A61L 2/16, C02F 1/32

(54) **Verfahren zur Bereitstellung von steriler Druckluft und/oder zumindest einer Flüssigkeit für medizinische Arbeitsgeräte**
Process for the preparation of sterile compressed air and/or of a liquid for medical implements
Procédé pour la préparation d'air comprimé aseptique et au moins un liquide pour outillages médicaux

(30) Priorität: 12.01.1990 AT 55/90
(43) Veröffentlichungstag der Anmeldung: 17.07.1991
(73) Patentinhaber: FOIDL, Leonhard, A-6263 Fügen (AT)
(72) Erfinder: FOIDL, Leonhard, A-6263 Fügen (AT)
(74) Vertreter: Hofinger, Engelbert, DDr.

(56) Entgegenhaltungen:
- EP-B- 0 019 622
- EP-B- 0 123 566
- DE-A- 3 221 350
- DE-C- 2 419 178

## Beschreibung

Die Erfindung betrifft ein Verfahren und eine Einrichtung zur Bereitstellung von steriler Druckluft und/oder von zumindest einer sterilen Flüssigkeit für medizinische, insbesondere dentalmedizinische Arbeitsgeräte mittels steriler Druchluft, wobei von einem Kompressor erzeugte Druckluft einen Aktivkohlefilter passiert.

Aus der EP-B-123 566 ist ein Verfahren zur Herstellung von steriler Luft für medizinische Zwecke beschrieben, bei dem atmosphärische Luft komprimiert, getrocknet und durch mehrere mit verschiedenen Aktivstoffen gefüllte Einrichtungen geleitet wird, die Wasser, Gas, wie Kohlenmonoxid, Staub und Bakterien entfernen. In einer bevorzugten Ausführung sind acht derartige Einrichtungen in Serie angeordnet. Die erzeugte sterile Luft dient zur Luftversorgung, beispielsweise in Krankenhäusern oder wird in Flaschen abgefüllt.

Die EP-B-19 622 beschreibt ein Verfahren zum Sterilhalten chirurgischer oder zahnärztlicher Handstücke, wobei gasförmiges und/oder flüssiges Medium auf seinem Förderweg zumindest zwei entkeimenden Mitteln ausgesetzt wird. Als entkeimende Mittel werden UV-Strahlen, Elektrodenanordnungen, Mikrowellen und/oder Oxidationsmittel verwendet.

Einem Kompressor zur Erzeugung von trockener Druckluft sind nach der DE-PS 24 19 178 ein Öl- und Wasserabscheider sowie ein Adsorptionsfilter nachgeschaltet. Die erzeugte Luft wird in einem Drucklufttank gespeichert.

Schließlich ist aus der DE-OS 32 21 350 ein Gerät zur Bereitstellung von sterilem Wasser bekannt, wobei vorgereinigtes Wasser aus einem Vorratsbehälter durch einen Aktivkohlefilter, einen Ionenaustauscher, einen Sterilfilter und einen der Entnahmestelle zugeordneten UV-Strahler geführt wird.

Die Erfindung hat es sich nun zur Aufgabe gestellt, ein Verfahren und eine Einrichtung zu entwickeln, die in möglichst einfacher Weise zur Bereitstellung von steriler Druckluft und/oder zumindest einer Flüssigkeit geeignet sind.

Erfindungsgemäß wird diese Aufgabe durch das Verfahren gemäß Anspruch 1 gelöst.

Derartige Flüssigkeiten sind beispielsweise Spül- oder Kühlwasser, physiologische Kochsalzlösung, Flüssigkeiten mit desinfiszierenden oder adstringierenden Zusätzen usw.

Bekannte Einrichtungen sind insbesondere deshalb so kompliziert und aufwendig, da beispielsweise das Spül- oder Kühlwasser durch Leitungswasser ergänzt wird bzw. andere Flüssigkeiten mit nicht steriler Umgebungsluft in Berührung kommen. Dies erfordert eine ständige Anwendung der sterilisierenden Einrichtungen und Mittel.

Das erfindungsgemäße Verfahren beruht auf der Überlegung, daß die Sterilität einer in einem gefüllten Vorratsbehälter anfangs sterilen Flüssigkeit, die mit zunehmender Entleerung durch nachströmende Flüssigkeit oder Luft verloren geht, dadurch erhalten werden kann, daß der Vorratsbehälter nicht ergänzt, sondern in diesen sterile Druckluft bis zu dessen vollständigen Entleerung eingebracht wird.

Anschließend wird der Vorratsbehälter entweder mit steriler Flüssigkeit befüllt bzw. ausgetauscht. Es sind daher weder eine fortlaufende sterilisierende Behandlung der Flüssigkeit, da deren einmal erreichte Sterilität nicht gefährdet wird, noch zusätzliche Einrichtungen für deren sterile Beaufschlagung erforderlich, da sterile Druckluft ohnedies erzeugt werden muß, und in ausreichenden Mengen zur Verfügung steht.

Von einem Kompressor für medizinische Zwecke erzeugte Druckluft (beispielsweise nach der genannten DE-PS 24 19 178) ist bereits bei Verlassen des Aktivkohlefilters im wesentlichen trocken und steril.

Eine bevorzugte Ausführung des Verfahrens, mittels der der Reinigungs-,Trocknungs- und Entkeimungsgrad optimiert werden kann, sieht vor, daß als Waschflüssigkeit konzentrierter Äthanol verwendet wird.

Durch die Beaufschlagung jedes Vorratsbehälters für Flüssigkeit mit steriler Druckluft ergibt sich eine konstruktiv besonders einfache Einrichtung zur Durchführung des Verfahrens wie in Anspruch 3 angegeben.

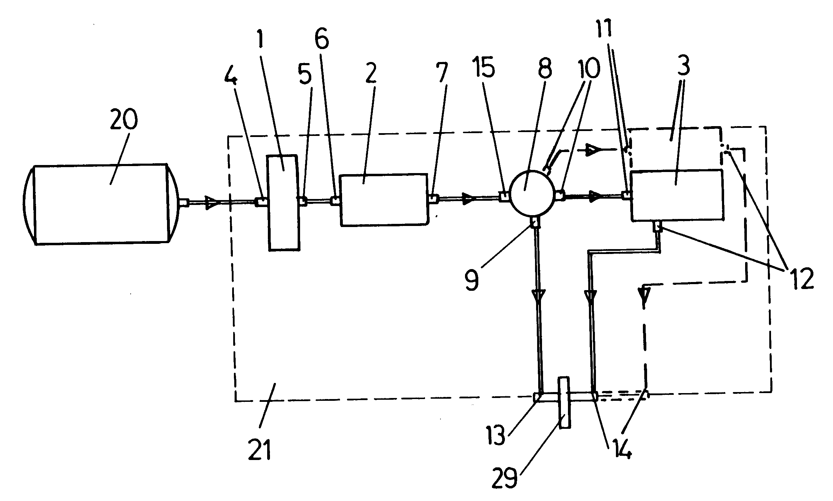

Nachstehend wird nun die Erfindung anhand der Figur der beiliegenden Zeichnung näher erläutert, die eine schematische Darstellung einer Einrichtung zur Bereitstellung von steriler Druckluft und/oder zumindest einer Flüssigkeit für medizinische Arbeitsgeräte zeigt.

Von einer außerhalb einer baulichen Einheit 21 angeordneten Druckluftquelle 20, insbesondere einem Kompressor, erstreckt sich eine Ausgangsleitung zum Einlaß 4 einer Aktivkohlefilterpatrone 1, deren Auslaß 5 über eine Leitung mit dem Einlaß 6 eines Behälters 2 verbunden ist, der eine hygroskopische, desinfiszierende Waschflüssigkeit, insbesondere konzentrierten Äthylalkohol, enthält. Die Patrone 1 und der Behälter 2 bzw. deren Inhalt sind austauschbar, wobei dies nicht näher dargestellt ist. Vom Auslaß 7 des Behälters für die Waschflüssigkeit wird die nunmehr hochsterile Druckluft über den Einlaß 15 einem Abzweiger 8 zugeführt. Dieser kann durch ein übliches Mehrwegeventil gebildet sein, es ist jedoch günstiger, den Ausgängen 9, 10 des Abzweigers 8 einzeln schaltbare Ventile zuzuordnen, sodaß auch beide gleichzeitig geöffnet werden können. Vom ersten Ausgang 9 des Abzweigers 8 führt eine Leitung zu einem Entnahmeauslaß 13 für die sterile Druckluft. Über jeden zweiten Ausgang 10 des Abzweigers 8 kann sterile Druckluft dem Einlaß 11 eines Vorratsbehälters 3 zugeführt werden, in dem eine Flüssigkeit, beispielsweise steriles Spül- oder Kühlwasser, physiologische Kochsalzlösung usw. enthalten ist. Jeder Vorratsbehälter 3 ist austauschbar, das heißt, die Verbindungen zu Einlaß 11 und Auslaß 12 sind lösbar; gegebenenfalls könnte jeder entleerte Vorratsbehälter 3 auch wieder mit der jeweiligen Flüssigkeit befüllt werden. Über die Auslässe 12 werden die in den Vorratsbehältern 3 von der sterilen Druckluft beaufschlagten Flüssigkeiten zu Entnahmeauslässen 14 gedrückt. Da die Flüssigkeit aus jedem Vorratsbehälter 3 vollständig ausgetragen wird und dabei ausschließlich mit der sterilen Druckluft in Berührung kommt, erübrigen sich weitere Maßnahmen und Mittel zur Sterilisierung der Flüssigkeiten. An die Entnahmeauslässe 13 und 14 sind Arbeitsgeräte 29 oder dergleichen anschließbar, bei deren Betrieb eine Anwesenheit von sterilen Flüssigkeiten vorteilhaft oder notwendig ist.

## Patentansprüche

1. Verfahren zur Bereitstellung von steriler Druckluft und/ oder von zumindest einer sterilen Flüssigkeit für medizinische, insbesondere dentalmedizinische Arbeitsgeräte mittels steriler Druchluft, wobei von einem Kompressor erzeugte Druckluft einen Aktivkohlefilter passiert, und die den Aktivkohlefilter verlassende Druckluft durch eine hygroskopische, desinfiszierende Waschflüssigkeit geleitet wird, worauf mit der sterilen Druckluft, die direkt zum Arbeitsgerät geführt wird, auch jede bevorratete sterile Flüssigkeit beaufschlagt wird.

2. Verfahren nach Anspruch 1, wobei als Waschflüssigkeit konzentrierter Äthanol verwendet wird.

3. Einrichtung zur Durchführung des Verfahrens nach Anspruch 1, wobei eine mit einem Druckluft-einlaß (4) versehene Aktivkohlefilterpatrone (1), ein Behälter (2) für hygroskopische, desinfiszierende Waschflüssigkeit und mindestens ein Vorratsbehälter (3) für eine Flüssigkeit zu einer Einheit (21) zusammengefaßt sind, in der der Auslaß (5) der Aktivkohlefilterpatrone (1) mit einem Einlaß (6) des Waschflüssigkeitsbehälters (2) verbunden ist, und an den Auslaß (7) des Waschflüssigkeitsbehälters (2) ein Abzweiger (8) angeschlossen ist, der einen mit einem Entnahmeauslaß (13) für Druckluft verbundenen ersten Ausgang (9) und pro Vorratsbehälter (3) einen mit dessen Einlaß (11) verbundenen zweiten Ausgang (10) aufweist, wobei der Auslaß (12) jedes Vorratsbehälters (3) mit einem Entnahmeanschluß (14) für die enthaltene Flüssigkeit versehen ist.

## Claims

1. A process for the preparation of sterile compressed air and/or at least one sterile liquid for medical and in particular dental-medical implements by means of sterile compressed air, wherein compressed air produced by a compressor passes through an activated carbon filter and the compressed air leaving the activated carbon filter is passed through a hygroscopic, disinfecting washing liquid, whereupon each stored sterile liquid is also acted upon by the sterile compressed air which is passed directly to the implement.

2. A process according to claim 1 wherein concentrated ethanol is used as the washing liquid.

3. Apparatus for carrying out the process according to claim 1 wherein an activated carbon filter cartridge (1) provided with a compressed air inlet (4), a container (2) for hygroscopic, disinfecting washing liquid and at least one storage container (3) for a liquid are combined together to form a unit (21) in which the outlet (5) of the activated carbon filter cartridge (1) is connected to an inlet (6) of the washing liquid container (2) and connected to the outlet (7) of the washing liquid container (2) is a branching means (8) which has a first outlet (9) connected to a take-off discharge (13) for compressed air and, for each storage container (3), a second outlet (10) connected to the inlet (11) thereof, wherein the discharge (12) of each storage container (3) is provided with a take-off connection (14) for the liquid contained therein.

## Revendications

1. Procédé de préparation d'air comprimé aseptique et /ou d'au moins un liquide aseptique pour instruments médicaux, en particulier en médecine dentaire, au moyen de l'air comprimé aseptique, procédé dans lequel l'air comprimé produit par un compresseur passe dans un filtre à charbon actif et dans lequel l'air comprimé quittant le filtre à charbon actif passe à travers un liquide hygroscopique, désinfestant, de lavage, sur quoi tout liquide aseptique stocké est également soumis à l'air comprimé aseptique, directement envoyé à l'instrument.

2. Procédé selon la revendication 1, dans lequel on emploie l'éthanol concentré comme liquide de lavage.

3. Dispositif pour la mise en oeuvre du procédé selon la revendication 1, dans lequel une cartouche filtrante à charbon actif (1), munie d'une entrée (4) pour l'air comprimé, un récipient (2) pour du liquide hygroscopique, désinfectant, de lavage et au moins un réservoir (3) pour un liquide sont regroupés pour donner un ensemble (21) dans lequel la sortie (5) de la cartouche filtrante à charbon actif (1) est reliée avec une entrée (6) du réservoir (2) de liquide de lavage et dans lequel, à la sortie (7) du réservoir (2) de liquide de lavage, est relié un organe de dérivation (8) qui présente une première sortie (9), reliée avec une sortie (13) de prélèvement de l'air comprimé et, par réservoir (3), une seconde sortie (10) reliée à son entrée (11), la sortie (12) de chaque réservoir (3) comportant un raccord de prélèvement (14) pour le liquide contenu.
